# EUROPEAN PATENT APPLICATION

(11) **EP 4 226 860 A1**
(43) Date of publication of application: **16.08.2023**
(21) Application number: 21877923.9
(22) Date of filing: 01.10.2021
(51) Int. Cl.: A61B 5/361, A61B 5/363, A61B 5/00

(54) **METHOD AND DEVICE FOR ANALYZING BIO-SIGNAL**

(30) Priority: 06.10.2020 KR 20200128484; 06.10.2020 KR 20200128979
(71) Applicant: Sky Labs Inc., Gyeonggi-do 13486 (KR)
(72) Inventor: LEE, Byung Hwan, Yongin-si Gyeonggi-do 16981 (KR); BAIK, Chang Hyun, Suwon-si Gyeonggi-do 16505 (KR); KIM, Chang Hyun, Seoul 05372 (KR); CHOI, Chang Woo, Uiwang-si Gyeonggi-do 16000 (KR); CHANG, Hyung Min, Yongin-si Gyeonggi-do 16843 (KR); JO, Sung Mi, Seoul 08706 (KR); KIM, Hae Na, Seongnam-si Gyeonggi-do 13466 (KR); KIM, Dae Sung, Yongin-si Gyeonggi-do 16928 (KR); JI, Yu Mi, Seongnam-si Gyeonggi-do 13607 (KR)
(74) Representative: Giuliano, Natalia
(86) International application number: PCT/KR2021/013538
(87) International publication number: WO 2022/075671

(57) **Abstract**

The present invention relates to a device for analyzing a bio-signal, and a method therefor, and the method for analyzing a bio-signal comprises the steps of: removing bio-signal noise generated by a sensor; estimating the signal quality of the bio-signal for a preset unit time; classifying the bio-signal for the preset unit time into an abnormal signal or a normal signal; and determining whether an abnormal state episode occurs and a duration time thereof on the basis of the signal quality of the bio-signal for a plurality of unit times and the result of the classification.

## Description

### Technical Field

The present invention relates to a method of analyzing biosignals and a device therefor. More particularly, the present invention relates to a method of determining an abnormal condition such as atrial fibrillation by analyzing a biosignal obtained from a sensor interfaced with a subject and a device therefor.

### Background Art

A condition in which the heart beats too slowly, too fast, or irregularly, i.e., an abnormal heartbeat, is called an arrhythmia. Atrial fibrillation (AF) is a type of arrhythmia, in which each atrium does not beat normally and tremors occur irregularly. As a symptom of atrial fibrillation, a rapid and irregular heartbeat is observed.

Atrial fibrillation is a relatively frequent arrhythmia. Atrial fibrillation is the most common symptom among patients with arrhythmia requiring treatment. About 33 % of all arrhythmia-related inpatients are patients with atrial fibrillation. Early and accurate prediction of frequently atrial fibrillation is of great medical significance.

Atrial fibrillation, in which the atria fail to contract normally and the atria tremble slightly, may cause symptoms such as difficulty in breathing and chest pain. In addition to these symptoms, there is an increased risk of more serious and dangerous arrhythmia. Atrial fibrillation prevents blood from being effectively pumped out of the heart, resulting in a variety of serious problems.

Compared to normal people, patients with atrial fibrillation have a 5 -fold increased risk of stroke and a 2-fold increased mortality rate. In addition, due to various heart disease-related complications caused by atrial fibrillation, the mortality rate of patients with atrial fibrillation is about twice that of normal people.

When a pulse rate increases rapidly due to sudden atrial fibrillation, cardiac output (the amount of blood pumped out when the heart contracts) rapidly decreases because there is not enough time to fill the heart with blood. In a normal heart, contraction of the atria accounts for about 30 % of the cardiac output, so a heart rate continuously increases to compensate for the insufficient cardiac output.

As a result of atrial fibrillation, a burden is applied to the heart, resulting in deterioration of the function of the heart and structural changes in the heart. As a result, heart failure may develop or worsen. In addition, when the heart is unable to contract normally due to atrial fibrillation, blood is congested in the heart and the risk of blood coagulation in the heart increases.

These blood clots that form within the heart travel through arteries and block blood vessels in the brain and other parts of the body. Accordingly, the risk of stroke or thromboembolism in patients with atrial fibrillation greatly increases.

When atrial fibrillation is accurately predicted and various atria pacing methods are considered, the possibility of preventing dangerous symptoms such as stroke or thromboembolism may be increased.

Therefore, to prevent these various dangerous diseases, a method of accurately determining and analyzing various abnormal physiological conditions in addition to atrial fibrillation described as an example by analyzing biosignals is required.

### Disclosure

### Technical Problem

Therefore, the present invention has been made in view of the above problems, and it is one object of the present invention to provide a method of accurately determining an abnormal condition episode by analyzing a biosignal obtained from a sensor interfaced with a subject and a device therefor.

### Technical Solution

In accordance with one aspect of the present invention, provided is a method of analyzing biosignals, wherein the method is performed using a device for analyzing biosignals and includes a step of removing noise from a biosignal generated by a sensor; a step of estimating signal quality of the biosignal of a preset unit time; a step of classifying the biosignal of a preset unit time as an abnormal signal or a normal signal; and a step of determining whether an abnormal condition episode has occurred and a duration time based on signal quality and abnormal signal classification results for biosignals of a plurality of unit times.

In one embodiment, the method includes a step of removing noise from a biosignal generated by a sensor; a step of estimating signal quality of the biosignal of a preset unit time; a step of classifying the biosignal of a preset unit time as atrial fibrillation (AF) or normal sinus rhythm (NSR); and a step of determining whether an AF episode has occurred and a duration time based on signal quality and atrial fibrillation classification results for biosignals of a plurality of unit times.

In one embodiment, the step of determining whether an AF episode has occurred and a duration time includes a step of determining that an AF episode has occurred when each of biosignals of consecutive unit times of more than a preset minimum number is classified as atrial fibrillation and signal quality is determined to be good; a step of determining, after occurrence of an AF episode, that the AF episode has ended when each of biosignals of a preset minimum number of consecutive unit times is determined as non-atrial fibrillation; and a step of calculating a time from start of the AF episode to end of the AF episode as a duration time.

The non-abnormal signal includes a case in which signal quality of a unit signal is good but normal sinus rhythm and a case in which signal quality is poor.

In the step of estimating signal quality of the biosignal, signal quality of the biosignal is estimated to be good or poor based on a preset machine learning algorithm based on correlation between characteristics of the biosignal and signal quality to be estimated.

In the step of classifying the biosignal as atrial fibrillation (AF) or normal sinus rhythm (NSR), the biosignal is classified based on a machine learning-based algorithm generated based on a correlation between characteristics of atrial fibrillation and normal sinus rhythm and characteristics of the biosignal.

The unit time is a predetermined period selected from 5 seconds, 10 seconds, and 20 seconds, and the preset number is a predetermined number selected from 2, 3, 4, and 5. However, the unit time and the preset number are not limited to the values.

In accordance with another aspect of the present invention, provided is a device for analyzing biosignals including a biosignal collection unit for collecting a biosignal generated by a sensor; a preprocessing unit for removing noise from the collected biosignal; a signal quality estimation unit for estimating signal quality of the biosignal of a preset unit time; a signal analysis unit for classifying the biosignal of a preset unit time as atrial fibrillation (AF) or normal sinus rhythm (NSR); and an episode determination unit for determining whether an AF episode has occurred and a duration time based on signal quality and AF classification results for biosignals of a plurality of unit times.

The episode determination unit determines that an AF episode has occurred when each of biosignals of consecutive unit times of more than a preset minimum number is classified as atrial fibrillation and signal quality is determined to be good; determines, after occurrence of an AF episode, that the AF episode has ended when each of biosignals of a preset minimum number of consecutive unit times is determined as non-atrial fibrillation; and calculates a time from start of the AF episode to end of the AF episode as a duration time.

The non-abnormal signal includes a case in which signal quality of a unit signal is good but normal sinus rhythm and a case in which signal quality is poor.

The signal quality estimation unit estimates signal quality of the biosignal as good or poor based on a preset machine learning algorithm based on a correlation between characteristics of the biosignal and signal quality to be estimated.

The signal analysis unit classifies the biosignal based on a machine learning-based algorithm generated based on a correlation between characteristics of atrial fibrillation and normal sinus rhythm and characteristics of the biosignal.

### Advantageous Effects

According to the present invention, by analyzing a biosignal obtained from a sensor interfaced with a subject, it can be accurately determined whether an abnormal condition episode has occurred. In addition, the present invention can help reduce hospitalization costs or reduce patient suffering.

### Description of Drawings

FIG. 1 is a block diagram for explaining a device for analyzing biosignals according to an embodiment of the present invention.
FIG. 2 is a flowchart for explaining an analysis method using a device for analyzing biosignals according to an embodiment of the present invention.
FIG. 3 includes vertical lines for explaining a case defined as a minimum unit AF episode and a case not defined as a minimum unit AF episode according to an embodiment of the present invention.
FIG. 4 includes vertical lines for explaining the occurrence time of AF episodes over time according to an embodiment of the present invention.
FIG. 5 includes vertical lines for additionally explaining AF episodes over time according to an embodiment of the present invention.
FIG. 6 includes vertical lines for explaining the combination of unit signals capable of defining an atrial fibrillation episode according to an embodiment of the present invention.

### Best Mode

Hereinafter, the present invention will be described in detail so that those skilled in the art can understand and easily practice the present invention through embodiments described with reference to the accompanying drawings.

In addition, in the following description of the present invention, a detailed description of known functions and configurations incorporated herein will be omitted when it may make the subject matter of the present invention unclear.

Prior to description of the present invention, in this specification, a unit signal refers to a signal collected for a preset unit time (e.g., 5 seconds, 10 seconds, 20 seconds, or the like).

An atrial fibrillation (AF) episode, which is an example of an abnormal signal among physiological abnormalities, means a clinically significant "event in which atrial fibrillation has occurred" based on a unit signal classified as atrial fibrillation (AF), as will be described later. That is, an atrial fibrillation episode is one event in which AF occurs and ends. Here, physiological abnormalities are not limited to atrial fibrillation, and may include hypertension, hypoxia, and high fever.

In addition, an abnormal condition episode that lasts for a preset minimum time (e.g., 15 seconds, 20 seconds, 30 seconds, or the like) is called a minimum unit abnormal condition episode.

In this specification, "a minimum unit abnormal condition (e.g., AF) episode may be defined" means that it may be determined whether a corresponding minimum unit is an abnormal condition episode.

In this specification, "defined as a minimum unit abnormal condition episode" means that a corresponding minimum unit is an abnormal condition episode.

In this specification, "may not be defined as a minimum unit abnormal condition episode" means that a corresponding minimum unit is not an abnormal condition episode.

In this specification, "a minimum unit abnormal condition episode may not be defined" means that it may not be determined whether a corresponding minimum unit is an abnormal condition episode.

### [Description of Symbols]

- 100:: DEVICE FOR ANALYZING BIOSIGNALS
- 110:: BIOSIGNAL COLLECTION UNIT
- 120:: PREPROCESSING UNIT
- 130:: SIGNAL QUALITY ESTIMATION UNIT
- 140:: SIGNAL ANALYSIS UNIT
- 150:: UNIT SIGNAL DETERMINATION UNIT
- 160:: EPISODE DETERMINATION UNIT

### Mode for Invention

Hereinafter, embodiments will be described in detail with reference to the accompanying drawings.

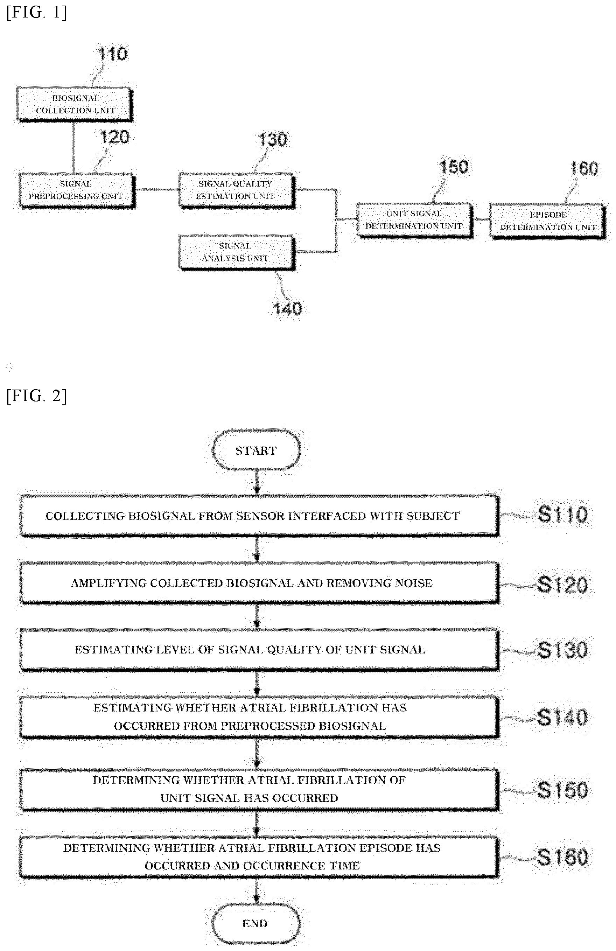

FIG. 1 is a block diagram for explaining a device for analyzing biosignals according to an embodiment of the present invention, and FIG. 2 is a flowchart for explaining an analysis method using a device for analyzing biosignals according to an embodiment of the present invention.

As shown in FIG. 1, a device 100 for analyzing biosignals includes a biosignal collection unit 110 and a processor P1. The processor P1 may include a preprocessing unit 120, a signal quality estimation unit 130, a signal analysis unit 140, a unit signal determination unit 150, and an episode determination unit 160.

Steps S100 to S160 included in the biosignal analysis method of FIG. 2 may be performed using the device for analyzing biosignals of FIG. 1.

In step S110, the biosignal collection unit 110 may include a sensor for measuring a biosignal, such as photoplethysmography (PPG), and obtain a biosignal such as a pulse wave signal from the sensor interfaced with a subject. The sensor may include a light source for radiating light to a subject and a detector for detecting light radiated by the light source and scattered or reflected from the surface of the subject or biological tissues such as blood vessels. For example, the PPG sensor may be a ring-type sensor that continuously collects and stores biosignals from blood vessels located in the finger.

The light source may include one or more light-emitting diodes (LEDs), laser diodes (LDs), or phosphors. In this case, when one or more light sources are configured, each light source may emit light of a different wavelength.

The detector may include one or more photo diodes, photo transistors (PTrs), or image sensors (e.g., CMOS image sensors). When one or more detectors are configured, the light sources may be placed at different distances from each detector. In addition, the pulse wave signal may mean a PPG signal, without being limited thereto.

In addition, in another embodiment, the biosignal collection unit 110 may receive a user's pulse wave signal from an external device by communicating with the external device. For example, the biosignal collection unit 110 may receive a biosignal such as user's pulse waves from the external device through Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, ultra-wideband (UWB) communication, Ant+ communication, WIFI communication, or Radio Frequency Identification (RFID) communication.

In step S120, the signal preprocessing unit 120 may include a filter, and the collected biosignal is amplified and noise is eliminated. Since the biosignal collected through the sensor may contain elements that make measurement inaccurate, such as noise, the signal preprocessing unit 120 removes elements that make measurement inaccurate.

In one embodiment, the signal preprocessing unit 120 may remove noise by exceptionally processing signals exceeding a predetermined threshold based on the size of a unit signal.

In one embodiment, the signal preprocessing unit 120 may extract a waveform of a signal and extract characteristics from the extracted waveform.

In step S130, the signal quality estimation unit 130 estimates the level of signal quality (hereinafter referred to as SQ) of the preprocessed unit signal.

In one embodiment, the signal quality estimation unit 130 operates based on a machine learning-based algorithm for quality estimation. For example, signal quality may be estimated based on the characteristics of a biosignal from which noise has been removed. As the similarity between the characteristics of a biosignal and a reference signal provided based on a machine learning-based algorithm generated based on correlation with signal quality to be estimated increases, signal quality may be estimated to be good. The range of signal quality levels is between 0 and 1. As the signal quality level approaches 1, the signal quality increases. As the machine learning-based algorithm, a pre-generated machine learning algorithm received from an external device through wired/wireless communication may be used.

The signal quality estimation unit 130 determines that the signal quality is good (SQ Good) when the estimated signal quality level is equal to or greater than a preset threshold, or determines that the signal quality is poor (SQ Poor) when the estimated signal quality level is less than a preset threshold.

In step S140, the signal analysis unit 140 estimates whether an abnormal signal, such as atrial fibrillation (AF), has occurred from the preprocessed biosignal. That is, the signal analysis unit 140 determines whether the preprocessed biosignal corresponds to an abnormal signal or normal sinus rhythm (NSR), which is a normal signal.

In one embodiment, the signal analysis unit 140 operates based on a machine learning algorithm for classifying abnormal signals. For example, based on a machine learning-based algorithm generated based on a correlation between the characteristics of atrial fibrillation and normal sinus rhythm and the characteristics of the biosignal, it may be determined whether the biosignal has atrial fibrillation characteristics or normal sinus rhythm characteristics. As the machine learning-based algorithm, a pre-generated machine learning algorithm received from an external device through wired/wireless communication may be used.

In step S150, the unit signal determination unit 150 determines whether an abnormal condition has occurred with respect to a unit signal based on the results of the signal quality estimation unit 130 and the signal analysis unit 140. For example, the unit signal determination unit 150 determines the reliability of the signal classification result of the signal analysis unit 140 based on the result of the signal quality estimation unit 130. That is, only when signal quality is good as a result of the signal quality estimation unit 130, the analysis result of the signal analysis unit 140 is trusted. As a result of the signal quality estimation unit 130, when signal quality is poor, the reliability of the signal itself is low, so the result of the signal analysis unit 140, which analyzes the signal with low reliability, is also not reliable, so the corresponding unit signal is determined as "unknown".

In summary, the unit signal determination unit 150 determines a biosignal analysis result as "unknown" due to low reliability when signal quality is low (poor signal). When signal quality is high (good signal), the unit signal determination unit 150 finally determines whether a biosignal is an abnormal signal or a normal signal according to the abnormal signal classification result of the signal analysis unit 140. For example, the unit signal determination unit 150 finally determines whether a biosignal corresponds to atrial fibrillation (AF) or normal sinus rhythm (NSR).

In step S160, an episode analysis unit 160 determines occurrence and duration of an abnormal condition episode during a certain period based on unit signals analyzed by the unit signal determination unit 150.

As an embodiment, an episode in which unit times determined as atrial fibrillation by the unit signal determination unit 150 last for a preset minimum time (e.g., 15 seconds, 20 seconds, 30 seconds, or the like) is defined as a minimum unit AF episode.

In addition, an AF episode has a minimum unit. Accordingly, "an AF episode may be defined" eventually leads to "a minimum unit AF episode, which is the minimum unit of an AF episode, may be defined".

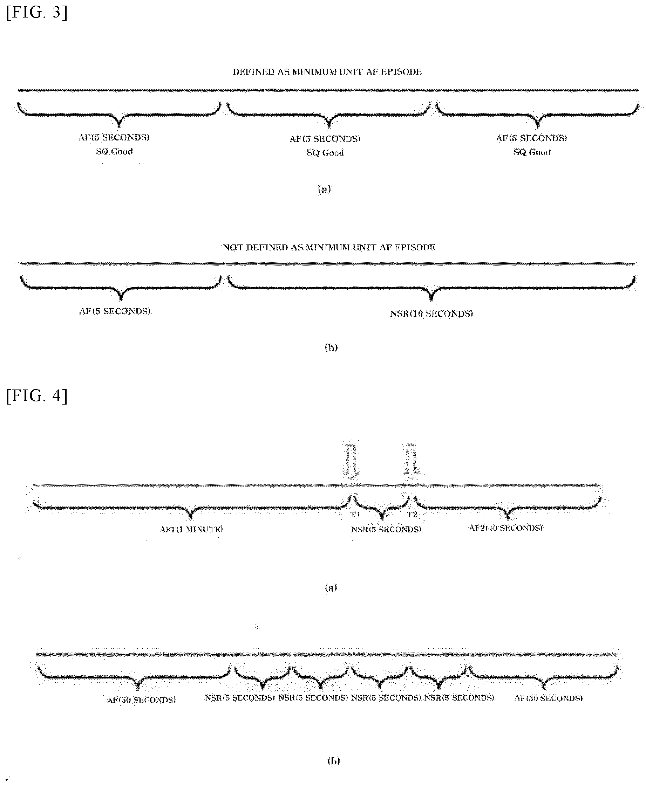

Hereinafter, some steps of the biosignal analysis method of FIG. 2 will be described in detail with reference to FIGS. 3 to 6. For convenience of description, atrial fibrillation analysis is described as an example, but the present invention is not limited thereto. Atrial fibrillation described in the following embodiments is an abnormal signal in another modified embodiment, and normal sinus rhythm is a normal signal in another modified embodiment.

FIG. 3 includes vertical lines for explaining a case defined as a minimum unit AF episode and a case not defined as a minimum unit AF episode according to an embodiment of the present invention. FIG. 3A includes vertical lines for explaining a case defined as a minimum unit AF episode, and FIG. 3B includes vertical lines for explaining a case not defined as a minimum unit AF episode. For example, assuming that the duration time of a unit signal is 5 seconds and the duration time of a minimum unit AF episode is 15 seconds, the duration time of the minimum unit AF episode is satisfied when the unit signal is three or more times. In addition, assuming that the duration time of a unit signal is 20 seconds and the duration time of a minimum unit AF episode is 100 seconds, the duration time of the minimum unit AF episode is satisfied when the unit signal is five or more times. In this way, the consecutive number of unit signals satisfying the duration time of the minimum unit AF episode is referred to as the minimum number of times.

As shown in FIG. 3A, a unit signal determined as atrial fibrillation continues three or more times in a row, and the episode analysis unit 160 determines an event that satisfies the minimum number of times or the minimum time as an AF episode.

As shown in FIG. 3B, when a unit signal determined as atrial fibrillation does not satisfy the minimum number of times or the minimum time, e.g., when a unit signal determined as NSR of 10 seconds lasts 2 times, i.e., 10 seconds, after lasting 1 time, i.e., 5 seconds, the corresponding event is not determined as an AF episode.

As such, the episode analysis unit (160 in FIG. 1) ignores a non-contiguous time and calculates an AF episode occurrence time when the non-contiguous time is less than a preset minimum time even when the AF episode is sensed in a non-continuous manner.

FIG. 4 includes vertical lines for explaining the occurrence time of AF episodes over time according to an embodiment of the present invention.

As shown in FIG. 4A, a case in which normal sinus rhythm (hereinafter referred to as NSR) occurs at a point T1 where an atrial fibrillation AF signal (AF1) lasts for 1 minute and an atrial fibrillation AF signal (AF2) lasts for 40 seconds at a point T2 where NSR lasts for 5 seconds is considered.

When the time between discontinuous points T1 and T2 of an AF signal (AF1) and an AF signal (AF2) is less than a preset minimum time (e.g., 20 seconds) for defining an episode, the episode analysis unit 160 ignores discontinuous time. In FIG. 4A, discontinuous time is ignored because the time between T1 and T2 is 5 seconds, which is less than a preset minimum time of 20 seconds. Accordingly, an AF episode occurrence time is 1 minute 45 seconds, which is the sum of an AF signal (AF1) duration time, an NSR duration time, and an AF signal (AF2) duration time.

Here, the reason why the episode analysis unit 160 ignores an NSR time less than the minimum time is to correct errors in sensing and analysis.

Specifically, when the signal analysis unit 140 determines that an arbitrary biosignal is NSR, the signal analysis unit 140 may correctly determine actual NSR as NSR and may misjudge actual AF as NSR.

However, after an AF episode has occurred, after normal sinus rhythm (NSR occurrence), which is a normal rhythm, is restored in a very short time, the probability of an AF episode occurring again is extremely low, so a biosignal determined as NSR for a very short time between AF episodes is ignored. Here, the very short time may be less than about 20 seconds as a meaningful time for clinical judgment. Accordingly, in FIG. 4A, the biosignal judged as NSR for a very short time of 5 seconds between AF1 and AF2 is determined to be an erroneous estimation of the actual AF generation signal as NSR, and the biosignal is ignored. In addition, in the present specification, a meaningful time or a very short time is not limited to 20 seconds or 5 seconds.

Here, when the probability that the signal analysis unit 140 correctly judges the actual NSR as NSR is P1, and the probability of misjudging the actual AF as NSR is P2, the signal analysis unit 140 may perform judgement by comparing a value obtained by multiplying the probability of an actual AF signal and the probability (P2) of misjudging an actual AF signal as an NSR signal and a value obtained by multiplying the probability that a signal is an actual NSR signal and the probability (P1) of correctly determining an actual NSR as NSR. Here, the probability that a signal is an actual AF signal and the probability that a signal is an actual NSR signal may be set in advance according to clinical statistics.

As another example, as shown in FIG. 4B, when an atrial fibrillation AF signal (AF1) lasts for 50 seconds, and an atrial fibrillation AF signal (AF2) lasts for 30 seconds after NSR lasts for 4 consecutive times for 5 seconds, since the time of NSR is more than 20 seconds, which is a preset minimum time, the episode analysis unit 160 analyzes the case of FIG. 4B into a total of two episodes: a first episode with an occurrence time of 50 seconds and a second episode with an occurrence time of 30 seconds that occurred 20 seconds later. In addition, the preset minimum time is not limited to 20 seconds, and may be set to other times such as 10 seconds or 30 seconds.

As described above, when the unit time NSR signal occurs 4 times in a row, the probability that 4 consecutive NSR signals were real AF is a value obtained by calculating an equation of (Probability of real AF signal × Probability (P2) of misjudging real AF signal as NSR signal)⁴. Since (P2)⁴ is a value close to 0, the value obtained by calculating the equation of (Probability of real AF signal × Probability (P2) of misjudging real AF signal as NSR signal)⁴ is also a value close to 0. In other words, it is interpreted that the probability that the actual AF signal is consecutively misjudged as NSR is very low. Accordingly, the probability of actual NSR occurring in at least one of four NSR signals is higher than the probability of incorrectly determining four signals as NSR in succession. Accordingly, when a predetermined number, e.g., 4 or more, of consecutive NSR signals occur, it may be determined that reliable NSR exists.

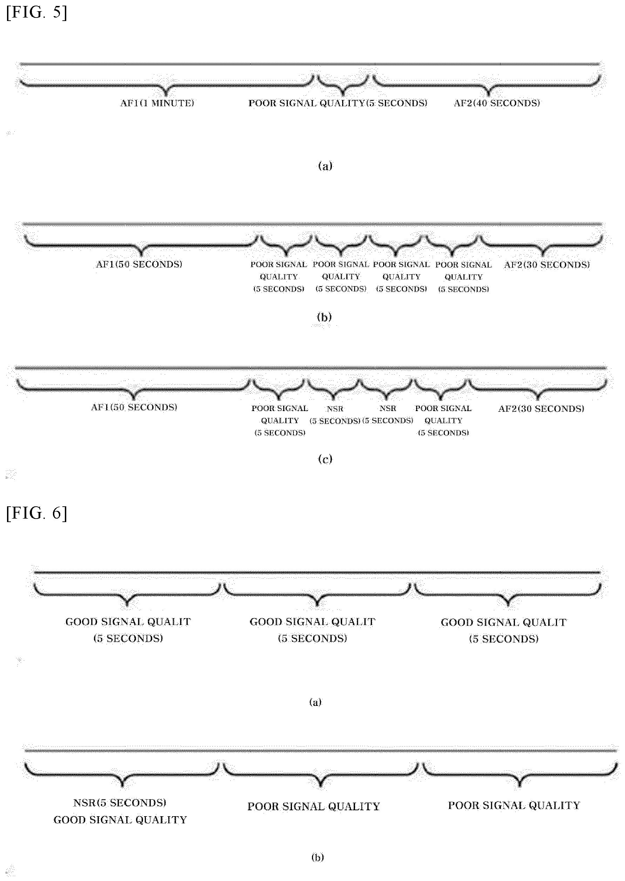

FIG. 5 includes vertical lines for additional explanation of AF episodes over time according to an embodiment of the present invention.

FIGS. 3 and 4 describe a case of good signal quality, but FIG. 5 describes a case in which poor signal quality is included.

As shown in FIG. 5A, a case in which an atrial fibrillation AF signal lasts for 1 minute, a unit signal (poor signal quality) determined to have poor signal quality lasts for 5 seconds, and then an atrial fibrillation AF signal lasts for 40 seconds is described.

Since poor signal quality between the AF signal (AF1) and the AF signal (AF2) occurred for 5 seconds, which is a very short time less than a minimum time of 20 seconds, the episode analysis unit 160 has no difficulty in determining that the AF signal is a signal generated within 5 seconds of poor signal quality. That is, a signal generated in a very short time (e.g., 5 seconds) with poor signal quality is determined according to the result of classification of the front and rear signals.

In the case of FIG 5A, the AF episode occurrence time is 1 minute 45 seconds, which is the sum of AF signal (AF1) duration time, poor signal quality duration time, and AF signal (AF2) duration time. More specifically, since the probability of an AF episode occurring again after an AF episode occurs and NSR occurs for a very short time is extremely low, even when it is impossible to determine whether it is AF due to signal failure between AF episodes, the very short time may be regarded as AF.

As another example, as shown in FIG. 5B, a case in which the atrial fibrillation AF signal (AF1) lasts for 50 seconds, the signal of poor signal quality continues 4 consecutive times for 5 seconds, and the atrial fibrillation AF signal (AF2) lasts for 30 seconds is described.

Since the time of poor signal quality is more than 20 seconds, which is a preset minimum time, the episode analysis unit 160 meaningfully interprets the 20-second period of poor signal quality, and analyzes the case of FIG 5B into two episodes: the first episode with an occurrence time of 50 seconds and the second episode with an occurrence time of 30 seconds that occurs 20 seconds later.

As another example, as shown in FIG. 5C, a case in which an atrial fibrillation AF signal (AF1) is generated and lasts for 50 seconds, a signal with poor signal quality lasts for 5 seconds, a normal sinus rhythm (NSR) with good signal quality lasts for 10 seconds, a signal with poor signal quality lasts for 5 seconds, and an atrial fibrillation AF signal (AF2) lasts for 30 seconds is described.

Signals with poor signal quality and normal sinus rhythm signals are called non-atrial fibrillation signals, and the non-atrial fibrillation signals are processed similarly to the signals with poor signal quality. Accordingly, since the time when the non-atrial fibrillation signal continuously occurs is 20 seconds or more, which is a preset minimum time, the episode analysis unit 160 meaningfully interprets the 20-second time period in which the non-atrial fibrillation signals occur consecutively, and analyzes the case of FIG. 5C into two episodes: the first episode with an occurrence time of 50 seconds and the second episode with an occurrence time of 30 seconds that occurs 20 seconds later.

To summarize, in the episode analysis unit 160, after occurrence of the minimum unit atrial fibrillation episode, at least consecutive minimum number of times (4 times), each unit signal is "signal quality good (SQ Good) & NSR" or "poor signal quality (SQ Poor)", the atrial fibrillation episode ends.

FIG. 6 includes vertical lines for explaining the combination of unit signals capable of defining an atrial fibrillation episode according to an embodiment of the present invention.

FIGS. 6A and 6B illustrate combinations of various unit signals included in the minimum unit of an AF episode having a duration time of 15 seconds.

As shown in FIG. 6A, when all unit signals included in a minimum unit have good signal quality, it is analyzed as "a minimum unit AF episode may be defined". Here, "a minimum unit AF episode may be defined" means "it may be determined whether a corresponding minimum unit is an AF episode". Conversely, "a minimum unit AF episode may not be defined" means "it may not be determined whether a corresponding minimum unit is an AF episode".

As shown in FIG. 6B, when at least one of unit signals included in the minimum unit is "good signal quality & normal sinus rhythm (NSR)", regardless of the signal quality level of the remaining unit signals or atrial fibrillation, it is analyzed as "the corresponding minimum unit is not an AF episode".

In summary,
1. "A minimum unit AF episode may be defined" means "it may be determined whether a corresponding minimum unit is an AF episode".
2. "Defined as a minimum unit AF episode" means "a corresponding minimum unit is an AF episode".
3. "May not be defined as a minimum unit AF episode" means "a corresponding minimum unit is not an AF episode".
4. "A minimum unit AF episode may not be defined" means "it may not be determined whether a corresponding minimum unit is an AF episode".

For example, 1) when the signal quality of all unit signals included in a minimum unit is good and all unit signals are determined to be AF, a corresponding minimum unit is determined as an AF episode. 2) When at least one of unit signals included in a minimum unit is "good signal quality & normal sinus rhythm", it is determined that a corresponding minimum unit is not an AF episode.

The apparatus described above may be implemented as a hardware component, a software component, and/or a combination of hardware components and software components. For example, the apparatus and components described in the embodiments may be achieved using one or more general purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions. The processing device may execute an operating system (OS) and one or more software applications executing on the operating system. In addition, the processing device may access, store, manipulate, process, and generate data in response to execution of the software. For ease of understanding, the processing apparatus may be described as being used singly, but those skilled in the art will recognize that the processing apparatus may include a plurality of processing elements and/or a plurality of types of processing elements. For example, the processing apparatus may include a plurality of processors or one processor and one controller. Other processing configurations, such as a parallel processor, are also possible.

The software may include computer programs, code, instructions, or a combination of one or more of the foregoing, configure the processing apparatus to operate as desired, or command the processing apparatus, either independently or collectively. In order to be interpreted by a processing device or to provide instructions or data to a processing device, the software and/or data may be embodied permanently or temporarily in any type of a machine, a component, a physical device, a virtual device, a computer storage medium or device, or a transmission signal wave. The software may be distributed over a networked computer system and stored or executed in a distributed manner. The software and data may be stored in one or more computer-readable recording media.

The methods according to the embodiments of the present invention may be implemented in the form of a program command that can be executed through various computer means and recorded in a computer-readable medium. The computer-readable medium can store program commands, data files, data structures or combinations thereof. The program commands recorded in the medium may be specially designed and configured for the present invention or be known to those skilled in the field of computer software. Examples of a computer-readable recording medium include magnetic media such as hard disks, floppy disks and magnetic tapes, optical media such as CD-ROMs and DVDs, magneto-optical media such as floptical disks, or hardware devices such as ROMs, RAMs and flash memories, which are specially configured to store and execute program commands. Examples of the program commands include machine language code created by a compiler and high-level language code executable by a computer using an interpreter and the like.

Although the present invention has been described with reference to limited embodiments and drawings, it should be understood by those skilled in the art that various changes and modifications may be made therein. For example, the described techniques may be performed in a different order than the described methods, and/or components of the described systems, structures, devices, circuits, etc., may be combined in a manner that is different from the described method, or appropriate results may be achieved even when replaced by other components or equivalents.

Therefore, other embodiments, other examples, and equivalents to the claims are within the scope of the following claims.

### Industrial Applicability

The present invention can provide a method of accurately estimating an abnormal condition episode by analyzing a biosignal obtained from a sensor interfaced with a subject and a device therefor.

## Claims

1. A method of analyzing biosignals, wherein the method is performed using a device for analyzing biosignals and comprises a step of removing noise from a biosignal generated by a sensor; a step of estimating signal quality of the biosignal of a preset unit time; a step of classifying the biosignal of a preset unit time as an abnormal signal or a normal signal; and a step of determining whether an abnormal condition episode has occurred and a duration time based on signal quality and classification results for biosignals of a plurality of unit times.

2. The method according to claim 1, wherein the step of determining whether an abnormal condition episode has occurred and a duration time comprises a step of determining that an abnormal condition episode has occurred when each of biosignals of consecutive unit times of more than a preset minimum number is classified as an abnormal signal and signal quality is determined to be good; a step of determining, after occurrence of an abnormal condition episode, that the abnormal condition episode has ended when each of biosignals of a preset minimum number of consecutive unit times is determined as a non-abnormal signal; and a step of calculating a time from start of the abnormal condition episode to end of the abnormal condition episode as a duration time.

3. The method according to claim 2, wherein the non-abnormal signal comprises a case in which signal quality of a unit signal is good but a normal signal and a case in which signal quality is poor.

4. The method according to claim 1, wherein, in the step of estimating signal quality of the biosignal, signal quality of the biosignal is estimated to be good or poor based on a preset machine learning algorithm based on correlation between characteristics of the biosignal and signal quality to be estimated.

5. The method according to claim 1, wherein, in the step of classifying the biosignal as an abnormal signal or a normal signal, the biosignal is classified based on a machine learning-based algorithm generated based on a correlation between characteristics of an abnormal signal and a normal signal and characteristics of the biosignal.

6. A device for analyzing biosignals, comprising:
a biosignal collection unit for collecting a biosignal generated by a sensor;
a preprocessing unit for removing noise from the collected biosignal;
a signal quality estimation unit for estimating signal quality of the biosignal of a preset unit time;
a signal analysis unit for classifying the biosignal of a preset unit time as an abnormal signal or a normal signal; and
an episode determination unit for determining whether an abnormal condition episode has occurred and a duration time based on signal quality and classification results for biosignals of a plurality of unit times.

7. The device according to claim 6, wherein the episode determination unit determines that an abnormal condition episode has occurred when each of biosignals of consecutive unit times of more than a preset minimum number is classified as an abnormal signal and signal quality is determined to be good; determines, after occurrence of an abnormal condition episode, that the abnormal condition episode has ended when each of biosignals of a preset minimum number of consecutive unit times is determined as a non-abnormal signal; and calculates a time from start of the abnormal condition episode to end of the abnormal condition episode as a duration time.

8. The device according to claim 7, wherein the non-abnormal signal comprises a case in which signal quality of a unit signal is good but a normal signal and a case in which signal quality is poor.

9. The device according to claim 6, wherein the signal quality estimation unit estimates signal quality of the biosignal as good or poor based on a preset machine learning algorithm based on a correlation between characteristics of the biosignal and signal quality to be estimated.

10. The device according to claim 6, wherein the signal analysis unit classifies the biosignal based on a machine learning-based algorithm generated based on a correlation between characteristics of an abnormal signal and a normal signal and characteristics of the biosignal.

11. The device according to claim 6, wherein the sensor is a photoplethysmography (PPG) sensor.
